# EUROPÄISCHE PATENTANMELDUNG

(11) **EP 2 269 649 A2**
(43) Veröffentlichungstag der Anmeldung: **05.01.2011**
(21) Anmeldenummer: 10009259.2
(22) Anmeldetag: 26.07.2005
(51) Int. Cl.: A61K 45/06, A61K 36/00, A23L 1/302, A23L 1/304, A23L 1/305

(54) **Ein Kapillar-Wirksystem enthaltende Zusammensetzungen mit anwendungsbezogener Differenzierbarkeit und deren Verwendung**

(30) Priorität: 26.08.2004 DE 102004041270
(62) Teilanmeldung aus: 05768354.2
(71) Anmelder: Merz Pharma GmbH & Co. KGaA, 60318 Frankfurt (DE)
(72) Erfinder: Beutler, Rolf, 64739 Höchst/ Humethroth (DE); Schmidt, Karl-Heinz, 72810 Gomaringen (DE); Funke, Bettina, 60316 Frankfurt/ Main (DE); Becker, Kirstin, 60439 Frankfurt/ Main (DE)
(74) Vertreter: Müller, Claudia

(57) **Zusammenfassung**

Die vorliegende Erfindung betrifft Zusammensetzungen mit einem Kapillar- Wirksystem mit anwendungsbezogener Differenzierbarkeit und synergistischem Potential für die Verwendung bei Säugern, vor allem Menschen.

Die Erfindung betrifft insofern eine Zusammensetzung, welche ein Kapillar- Wirksystem K aufweist, enthaltend wahlweise ein Kapillar - Aktivatorsystem KA aus Blutfluss steigernden , Gefäßwand stabilisierenden und/oder Gefäß erweiternden Mitteln; und / oder ein Kapillar- Protektorsystem KP, umfassend Mittel zur Endothelstabilisierung, zum Lipoproteinschutz, sowie zur Leukozyten- / Thrombozytenstabilisierung; und /oder ein Kapillar- Energieversorgungssystem KE, umfassend Redoxsysteme und Cofaktoren bei der Energiebereitstellung sowie Energieträger.

Dieses System K wird mit einem selektiven Wirksystem S kombiniert.

Die Erfindung betrifft ferner die Verwendung derartiger Zusammensetzungen, insbesondere systemisch bzw. enteral, vor allem zur selektiven Beeinflussung von z. B. strukturellen Veränderungen, funktionellen Störungen der betreffenden Zielorgane (Haare, Haut, Cerebrum, Skelett, Muskeln, Gastro-Intestinaltrakt, Augen) als Supplemente (Nahrungsergänzungsmittel, Ergänzende bilanzierte Diät, Diätetikum), oder pharmazeutisches Mittel.

## Beschreibung

### Gegenstand der Anmeldung

Die vorliegende Erfindung betrifft Zusammensetzungen mit einem Kapillar- Wirksystem mit anwendungsbezogener Differenzierbarkeit und synergistischem Potential für die Verwendung bei Säugern, vor allem Menschen.

Die Erfindung betrifft insofern eine Zusammensetzung, welche ein Kapillar- Wirksystem K aufweist, enthaltend wahlweise ein Kapillar - Aktivatorsystem KA aus Blutfluss steigernden , Gefäßwand stabilisierenden und/oder Gefäß erweiternden Mitteln; und / oder ein Kapillar- Protektorsystem KP, umfassend Mittel zur Endothelstabilisierung, zum Lipoproteinschutz, sowie zur Leukozyten- / Thrombozytenstabilisierung; und /oder ein Kapillar- Energieversorgungssystem KE, umfassend Redoxsysteme und Cofaktoren bei der Energiebereitstellung sowie Energieträger.

Dieses System K wird mit einem selektiven Wirksystem S kombiniert.

Die Erfindung betrifft ferner die Verwendung derartiger Zusammensetzungen - insbesondere systemisch bzw. enteral -, vor allem zur selektiven Beeinflussung von z. B. strukturellen Veränderungen, funktionellen Störungen der betreffenden Zielorgane (Haare, Haut, Cerebrum, Skelett, Muskeln, Gastro- Intestinaltrakt, Augen) als Supplemente (Nahrungsergänzungsmittel, Ergänzende bilanzierte Diät, Diätetikum), oder pharmazeutisches Mittel.

### Stand der Technik

Zur Versorgung einzelner Organe werden Arzneimittel eingesetzt. Diese zielen vor allem auf die Organ spezifische Wirkung einzelner Substanzen ab, wobei durch diese selbst über Transport / Verweilzeit entschieden wird.

Daneben sind auch insbesondere Supplemente wie Nahrungsergänzungsmittel (Mikronährstoffpräparate) bekannt. Dabei handelt es sich zum einen um Produkte mit einer Reihe unterschiedlichster Inhaltsstoffe, mit welchen möglichst breit verschiedene Bedürfnisse des Organismus abgedeckt werden sollen (z. B. Multivitaminpräparate). Dabei bleibt unklar, ob tatsächlich ein derartig breites Wirkungsspektrum aktiv vorhanden ist, oder ob beabsichtigte Wirkungen nicht gegenseitig aufgrund der Fülle an unterschiedlichen Wirkgruppen aufgehoben werden.

Aus diesem Grund werden auch Produkte eingesetzt, welche jeweils nur einen Wirkstoff für eine bestimmte Anwendung besitzen (z. B. Monovitaminpräparate). Damit muss für die jeweiligen unterschiedlichen Substanzen auch jeweils ein entsprechendes System bereitet werden, damit der beabsichtigte Zweck auch erreicht wird. Dieses kann auch von einer bestimmten Darreichungsform abhängen. So beschreibt z. B. die DE 43 26 698 C2 die Verwendung einer Vitamin B6 / B12 / Folsäure- Kombination insbesondere als Injektion zur Prophylaxe / Therapie von Arteriosklerose. Selbige Kombination wird nach der DE 43 26 675 C2 zur Vorbeugung von Nervendegenerationserkrankungen und Altersdementia eingesetzt.

In der DE 100 22 510 A1 werden Mittel, enthaltend Folsäure, 5-Methyltetrahydrofolsäure und / oder 5-Methyltetrahydrofolsäurepolyglutamat als Nahrungsergänzungsmittel beschrieben.

In der DE 102 06 159 A1 sind Mittel, enthaltend spezielle Kombinationen aus Folsäure, Vitamin B6, B12 und deren Verwendung zur Senkung von Homocysteinspiegeln offenbart.

In der DE 100 35 088 C2 wird ein Vitamin C Präparat offenbart, das in einer besonderen Form, nämlich als Bläschen oder Kügelchen mit einem Durchmesser von 0,2 mm, vorliegt.

Die DE 198 58 372 A1 betrifft ein Vitaminpräparat, umfassend ein Trägermaterial aus einem Zucker in Form eines Pulvers, auf das ein Vitamin aufgebracht ist, sowie ein Bindemittel in kristalliner, agglomerierter Form. Durch diese Form des Bindemittels soll es zu einem schnellen Auflösen beim Kontakt mit einer geringen Flüssigkeitsmenge kommen.

Aus der DE 100 16 313 A1 sind pharmazeutische Zubereitungen zur oralen oder peroralen Applikation bekannt, die in Form eines Gels mit einer Retardierung der Wirkstofffreisetzung im Magen-Darm Trakt freigesetzt werden.

Die EP B 0 531 155 beschreibt die Verwendung von Johannisbeerensaft ohne Obstkernöl oder ungesättigte Fettsäuren zur Förderung der Monoamin- Oxidase Inhibition.

DE GM 201 16 346 betrifft ein Mikronährstoffkombinationsprodukt mit bestimmten Mengen an Vitaminen wie 350 bis 700 mg Vitamin C, etc. und Carotinoiden (2-10 mg), welche in getrennten Verabreichungsformen wie Vitamin C- Filmtablette + getrennte Carotinoid- Kapseln vorliegen. Damit soll Mangelerscheinungen begegnet und insbesondere auch keine Verstärkung von Nebenwirkungen bei gleichzeitiger Arzneimittelgabe hervorgerufen werden.

In der EP B 0 796 080 wird ein spezielles Kombinationspräparat zur Förderung des Haar- und Haut-/Nagelwachstums beschrieben, welches ebenfalls Vitamin C in relativ hohen Mengen (15,61%) und weitere Vitamine /Inhaltsstoffe in bestimmten Mengenanteilen aufweist.

EP B 1 001 685 betrifft Nahrungszusammensetzungen, enthaltend Kohlenhydrate, Fette und Eiweiß sowie insbesondere eine bestimmte Menge an Methionin/Cystein.

In der GB A 2 292 522 wird ein Multivitaminpräparat beschrieben, welches Proteine sowie eine Mischung aus Vitaminen sowie Emulgatoren aufweist und zur Herbeiführung eines normal funktionierenden Immunsystems geeignet ist.

In der Roten Liste® 2004 wird unter Präparat Nr. 84166 ein Dragee mit Vitaminen wie Vitamin A, C, Calciumpanthothenat etc. sowie u.a. Methionin beschrieben.

Wie hieraus ersichtlich, werden entweder oder nur einzelne Wirkstoffe eingesetzt oder eine Mehrzahl hiervon kombiniert, wobei dann insbesondere auch galenische Maßnahmen hinsichtlich der Wirkstoffe selbst oder bzgl. der damit verbundenen Begleitmaterialien ergriffen werden, um die Stabilität, das Auflösungsvermögen oder die Freisetzungsgeschwindigkeit zu regulieren. Derartige Maßnahmen beziehen sich also auf die physikalischen Eigenschaften der Darreichungsform selbst und sind weitest gehendst somit auch von den eingesetzten Wirkstoffen abhängig. Damit müssen je nach Wirkstoff immer neue oder abgewandelte Applikationsformen entwickelt werden, wobei diese entweder nur auf einen Wirkstoff bzw. eine spezielle Wirkstoffgruppe oder eine große Menge unterschiedlicher Wirkstoffe ohne selektive Differenzierbarkeit abgestellt werden, d.h. ohne dass ein generelles Anwendungskonzept vorliegt.

### Aufgabe vorliegender Erfindung

Aufgabe vorliegender Erfindung ist es daher, den oben beschriebenen Mängeln abzuhelfen. Es soll ein Konzept entwickelt werden, wodurch mit Hilfe eines einzigen Systems - also Moduls - ein multidifferenzierbarer Wirkstofftransport auf insbesondere systemisch bzw. enteralem Weg zu einer rationalen, schnellen und vor allem selektiven Übertragung von Wirkstoffen auf dafür bestimmte Ziele bzw. Zielorgane erfolgt, ohne dass für die jeweiligen Wirkstoffe jeweils eine eigene galenische Formulierung entwickelt werden müsste.

### Lösung der Aufgabe

Diese Aufgabe wird erfindungsgemäß dadurch gelöst, dass man neben einem spezifischen Wirksystem S ein multidifferenzierbares Kapillar- Wirksystem K bereitstellt , umfassend wenigstens ein System, ausgewählt aus einem Kapillar- Aktivatorsystem KA, einem Kapillar- Protektorsystem KP und / oder einem Kapillar-Energieversorgungssystem KE.

Erfindungsgemäß wird somit ein generell einsetzbares Modul erhalten, das auf vielfältige Weise je nach gewünschtem Zielorgan eingesetzt werden kann.

Mit Hilfe eines derartigen Systems ist es möglich, eine anwendungsbezogene erhöhte Effizienz insbesondere aufgrund eines schnelleren und rationaleren Transports durch Einsatz des beschriebenen Kapillar Wirksystem K zu erreichen. Dadurch kann es auch ermöglicht werden, dass die aktiven Mittel rationaler am Zielort (Zielorgan) wirken, so dass auch die Anwendungsdosis bei systemisch bzw. enteraler Verabreichung, erniedrigt werden kann.

Insgesamt wird so Wirksamkeit in Abhängigkeit vom Ziel(Organ) multifunktionell - selektiv gesteuert. Damit ist vor allem eine Beeinflussung oder Behandlung von Haut/ -Anhangsgebilde (Haar, Nägel), also des keratinösen Systems, oder auch des immunologischen, ferner des cerebralen sowie des skeletösen Systems, des Muskel- des Gatrointestinaltraktes oder des Auges wirksam und unabhängig voneinander möglich. Erfindungsgemäß wird somit erstmals ein System zur Verfügung gestellt, das nicht auf die besondere Be- und Verarbeitung der speziellen aktiven Wirkstoffe ausgerichtet ist, sondern vielmehr unabhängig davon eine verbesserte Versorgung bzw. Beeinflussung bedürftiger Zielorgane unabhängig voneinander durch Aktivierung des Kapillarsystems ermöglicht.

Die erfindungsgemäße Zusammensetzung ist somit von der Darreichungsform unabhängig und ist vorzugsweise zur enteralen, systemischen, insbesondere zur oralen Anwendung konzipiert.

### Beschreibung der Erfindung

Die Erfindung betrifft insbesondere eine Zusammensetzung mit einer anwendungsbezogenen Wirkstoffdifferenzierbarkeit, die dadurch gekennzeichnet ist, dass sie
I.) ein Kapillar- Wirksystem K aufweist, das ein oder mehrere Systeme umfasst, ausgewählt aus einem
   a) Kapillar - Aktivatorsystem KA, umfassend ein oder mehrere Mittel, die ausgewählt sind aus einer oder mehreren Gruppen aus
      KA 1) Blutfluss steigernden Mitteln;
      KA 2) Gefäßwand stabilisierenden Mitteln;
      KA 3) Gefäß erweiternden Mitteln;
      und / oder einem
   b) Kapillar-Protektorsystem KP, umfassend ein oder mehrere Mittel, die ausgewählt sind aus einer oder mehreren Gruppen aus
      KP 1) Mitteln zur Endothelstabilisierung;
      KP 2) Mitteln zum Lipoproteinschutz;
      KP 3) Mitteln zur Leukozyten- / Thrombozytenstabilisierung;
      und / oder einem
   c) Kapillar- Energieversorgungssystem KE, umfassend ein oder mehrere Mittel, die ausgewählt sind aus einer oder mehreren Gruppen aus
      KE 1) Redoxsystemen bei der Energiebereitstellung;
      KE 2) Cofaktoren bei der Energiebereitstellung;
      KE 3) Energieträgern;
      und
II.) ein spezifisches Wirksystem S, ausgewählt aus Mitteln der Gruppe, umfassend Haut- und -Anhangsgebilde beeinflussende Mittel S1, cerebral wirksame Mittel S2, immunologisch wirksame Mittel S3, Knochen beeinflussende Mittel S4, Muskelwirksame Substanzen S5, den Gastrointestinaltrakt beeinflussen de Substanzen S6, das Auge beeinflussende Mittel S7,
   sowie
III.) 0,1 bis 90% Zusatzstoffe, insbesondere galenisch /pharmazeutische Zusatzstoffe, enthält.

Mit Hilfe einer solchen Zusammensetzung, insbesondere für die systemisch bzw. enterale Verwendung, ist es möglich, insbesondere bei Kombination mit bestimmten Substanzen oder Substanzgruppen (Wirkstoffe, Nährstoffe, pharmazeutische Wirkstoffen) jeweils eine anwendungsbezogene Wirkung zu erzielen, die gegenüber den beschriebenen Multivitamin- Nährstoffpräparaten sehr viel selektiver / effektiver und gegenüber monofunktionellen Produkten sehr viel effektiver ist, da durch das Kapillar -Wirksystem K eine erhöhte Kapillaraktivität und eine gesteigerte Bedarfslage entwickelt wird. Dadurch können die Wirksubstanzen in höherem Ausmaß spezifisch transportiert und eine verbesserte Aktivität bewirkt werden. Dies war insbesondere deshalb überraschend, weil zwar einzelne Komponenten des Kapillar- Wirksystems K an sich bekannt waren, jedoch nicht erwartet werden konnte, dass mit der speziellen Kombination gezielt unterschiedliche Organe selektiv bei erhöhter Bedarfslage angesteuert werden können.

Dabei können unterschiedlichste Darreichungsformen eingesetzt werden. Hierzu gehören insbesondere orale Mittel wie Dragees, Tabletten, Kapseln, etc. Die erfindungsgemäße Zusammensetzung ist somit für die systemisch bzw. enterale Anwendung geeignet. Überraschender Weise ist es hierbei nicht erforderlich, eine besondere Darreichungsform in Abhängigkeit von dem oder den spezifischen Wirkstoffen zu entwickeln, da aufgrund des Wirksystems K allgemein eine Verbesserung der Effizienz unterschiedlicher spezieller Wirkstoffe des Systems S möglich ist.

Die speziellen Wirkstoffe werden dabei vorzugsweise ausgewählt aus
S 1) Keratin, insbesondere Haut - und -Anhangsgebilde beeinflussenden Substanzen;
S 2) Cerebral wirksamen Substanzen;
S 3) Immunologisch wirksamen Substanzen;
S 4) Knochen beeinflussenden Substanzen;
S 5) Muskelwirksamen Substanzen;
S 6) Gastrointestinal beeinflussenden Substanzen;
S 7) Augen wirksamen Mittel.

Die Zusammensetzungen eignen sich daher insbesondere zur Beeinflussung von strukturellen Veränderungen, Störungen der betreffenden Zielorgane und sind zur systemischen bzw. enteralen, vor allem systemischen oralen Applikation geeignet.

In der erfindungsgemäßen Zusammensetzung können die einzelnen Systeme der Gruppe K in einer Menge von 0,001 bis 80 %, bevorzugt 0,001 bis 50%, insbesondere 0,01 bis 25%, bezogen jeweils auf KA, KP, oder KE, wobei die Summe an KA + KP + KE bis 98%, vorzugsweise bis 80% betragen kann; 0 bis 90%, vorzugsweise 0 bis 65%, insbesondere 0,01 bis 45% Zusatzstoffe und 0,001 bis 50%, insbesondere 0,001 bis 25% spezifisches System S vorliegen.

Die Mengenangaben beziehen sich auf das Gesamtgewicht der Zusammensetzung.

Mit der erfindungsgemäßen Zusammensetzung werden somit 2 Systeme in besonders wirksamer Weise zusammen mit Zusatzstoffen miteinander verknüpft, nämlich das allgemeine Kapillar- Wirksystem K, mit Kapillaraktivator KA, Kapillar-protektor KP und / oder Kapillar- Energieversorgungssystem KE , und das spezifische Wirksystem S, mit den entsprechenden Wirksubstanzen für Haut/Haare/Nägel, Knochen, Muskel, Verdauung, Auge, Immunsystem, Gehirn.

Das Zusammenwirken beider Systeme K +S ist in nachfolgendem Schema dargestellt:

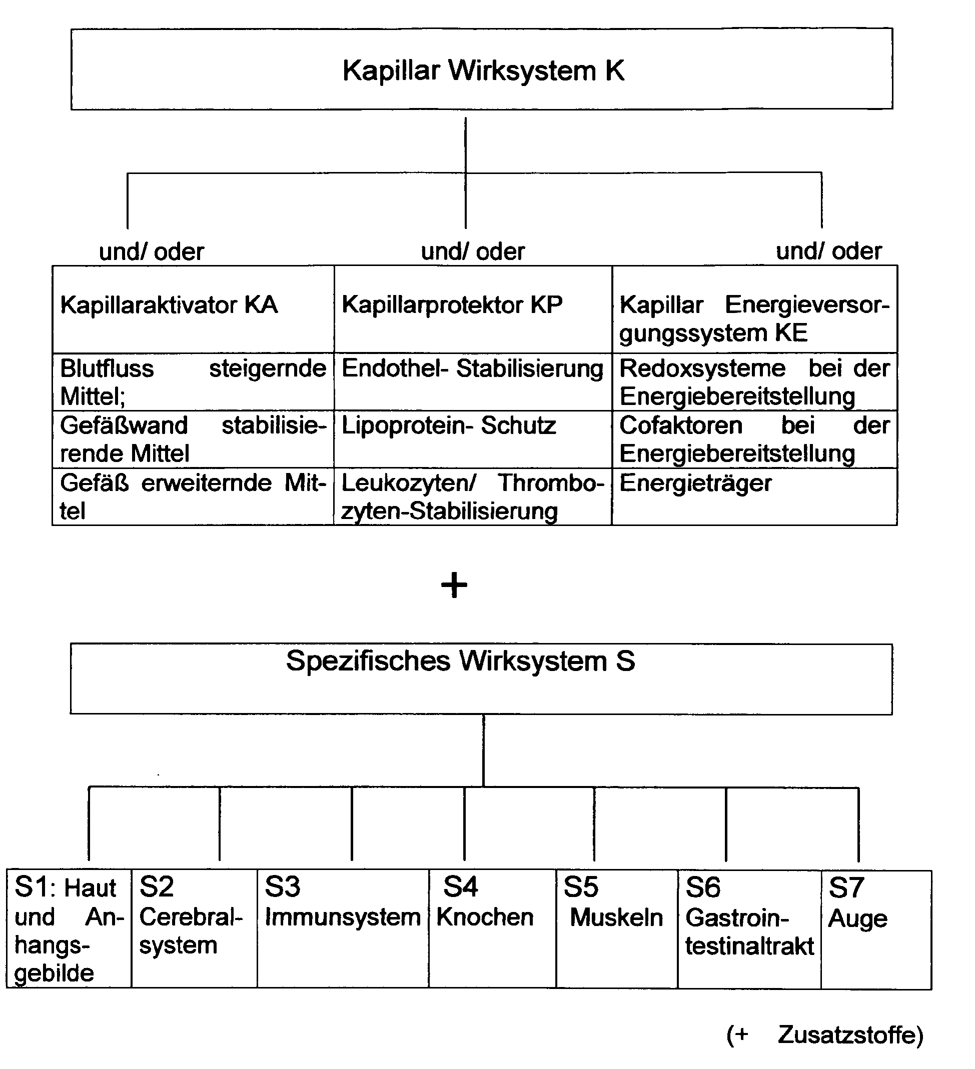

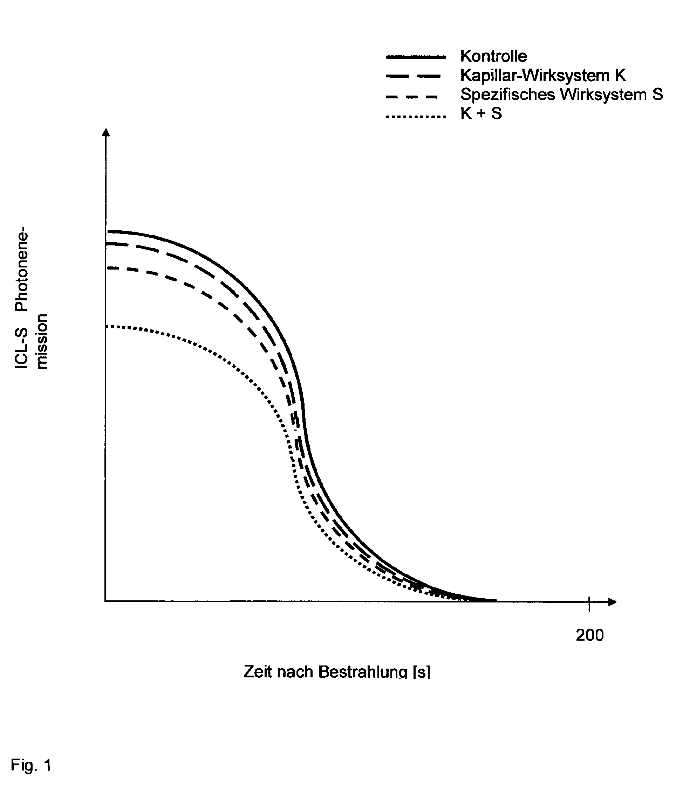

### Beschreibung der Fig. 1

In Fig. 1 ist die besondere Wirksamkeit einer erfindungsgemäßen Zusammensetzung gegenüber Vergleichszusammensetzungen anhand einer ICL- S-Messung aufgezeigt.

### Nähere Erläuterung und bevorzugte Ausführungsformen der Erfindung

### I) Kapillar- Wirksystem K

Die erfindungsgemäße Zusammensetzung umfasst ein Kapillar- Wirksystem K, das eine oder mehrere, also wenigstens ein System, ausgewählt aus I a), I b), I c) wie beschrieben aufweist.

Die Zusammensetzung kann also ein Kapillar- Aktivatorsystem KA oder ein Kapillar- Protektorsystem KP oder ein Kapillar - Energieversorgungssystem KE oder Kombinationen aus 2 oder 3 Vertretern dieser Gruppe aufweisen. Insbesondere ist ein Kapillar- Aktivatorsystem KA enthalten.

Vorzugsweise umfasst das Kapillar -Wirksystem K ein System KA zusammen mit einem System KP. In einer weiteren bevorzugten Ausführungsform liegt eine Kombination aus KA, KP und KE oder aus KA und KE vor.

Dabei hat sich gezeigt, dass das Kapillar -Aktivatorsystem KA aufgrund blutzirkulativer Eigenschaften eine Aktivierung dieses Systems bedingt. Das System KA ist somit agonistisch wirksam und kann selbst überadditive, bis gegebenenfalls synergistische Effekte auf bei Kombination von Mitteln der Gruppe KA 1) + KA 2); KA 1) + KA 3); KA 2) + KA 3) oder KA 1)+2)+3) auslösen.

Das System KP führt insbesondere zu einer Endothelstabilisierung, einem Lipoproteinschutz sowie einer Leukozyten- / Thrombozytenstabilisierung, wodurch zusätzlich Schadwirkungen z. B. durch Oxidantien zurückgedrängt und damit die Regeneration des Endothels ermöglicht wird. System KP ist somit insgesamt als antagonistisch zu bezeichnen.

Das System KE kann schließlich zu einer zusätzlichen Verstärkung durch agonistisches Wirken führen.

Insbesondere kann durch Kombination aus agonistischem System KA (KA1/2/3) und dem antagonistischen System KP oder mit dem antagonistischen System KP und System KE oder durch Kombination von KA/KP/KE, mit einem gewünschten Wirksystem S auch ein überadditiver bis gegebenenfalls synergistischer Effekt erzielt werden. Dieser lässt sich anhand geeigneter Methoden (ICL-S- Messung, Bildgebende Verfahren (Kapillarangiographie, Thermographie), Oximetrie) bzw. der Histaminreagibilität nachweisen. Die besondere Wirksamkeit einer erfindungsgemäßen Zusammensetzung gegenüber Vergleichszusammensetzungen ist anhand eines nachfolgenden Anwendungsbeispiels und in Fig. 1 mittels ICL-S-Messung aufgezeigt.

Die für die einzelnen Systeme KA, KP, KE einzusetzenden Mittel mit den genannten Eigenschaften wie angegeben sind dem Fachmann an sich bekannt. Beispielhaft können die folgenden, insbesondere auch bevorzugten Substanzen genannt werden:
Kapillar - Aktivatorsystem KA:
   Weinblätter-Extrakt, Rutin, Aescin, Roßkastanien-Extrakt, Mäusedorn-Extrakt, Eisencarbonat / -fumarat / -diphosphat / -lactat / -saccharat / -gluconat / -sulfat / - citrat (z. B. insbesondere in einer Menge entsprechend 6 mg Eisen (allein oder in Kombination)), Ginkgo-Extrakt, insbesondere NO- Releaser wie - Arginin und dessen Salze wie L-Arginin-Hydrochlorid . Letzteres bzw. deren Lieferanten, allein oder in Kombination, können insbesondere in einer Menge entsprechend 250 mg L-Arginin vorliegen.
Kapillar -Protektorsystem KP:
   Tocopherol / -acetat (Vitamin E), Anthocyane, α-Liponsäure, Folsäure (B₉), Pyridoxin HCL (B₆), Cyanocobalamin (B₁₂), Borretschöl, Leinöl, Traubenkernöl, schwarzes Johannisbeersamenöl, Nachtkerzenöl, Lachsöl, Schwarzkümmelöl (Lieferanten für Ω-3- und Ω-6-Fettsäuren).
Kapillar- Energieversorgungssystem KE:
   Nicotinsäure (Niacin) und dessen geeignete Derivate wie insbesondere Nicotinsäureamid (B₃), Magnesiumoxid / -citrat / -carbonat / -chlorid / gluconat / - phosphat / -lactat / -sulfat, Riboflavin (B₂), L- Carnitin, Q₁₀, Coffein, Glucose. Besonders bevorzugte Ausführungsformen sind **dadurch gekennzeichnet, dass** sie ein Kapillar Aktivatorsystem KA und ein Kapillar-Protektorsystem KP oder ein Energieversorgungssystem KE; oder auch solche, die neben dem Kapillar - Aktivatorsystem KA ein Kapillar- Protektorsystem KP und ein Kapillar- Energieversorgungssystem KE aufweisen.
   Ganz besonders bevorzugte Mittel für das System KA sind Arginin, und dessen Salze (z. B. insbesondere wie beschrieben), Ginkgo- Extrakt oder Kombinationen hiervon; für das System KP Folsäure, Cyanocobalamin, Pyridoxin HCL (B₆), Tocopherylacetat oder Kombinationen hiervon; und für das System KE Nicotinsäure (Niacin) und dessen geeignete Derivate wie insbesondere Nicotinsäureamid, Magnesiumoxid; Co Enzym Q10, Riboflavin oder Kombinationen hiervon.
   Bevorzugt beträgt die Menge an System KA, KP, KE jeweils 0,001 bis 80 %, bezogen auf das Gesamtgewicht der Zusammensetzung. Insbesondere bevorzugt sind Mengen von 0,01 bis 50%, vor allem 0,01 bis 35%.
   Alle Mengen- und Prozentangaben beziehen sich auf das Gewicht der Zusammensetzung, sofern nicht anders angegeben.
   Ganz besonders bevorzugt sind weiterhin Zusammensetzungen der beschriebenen Art, worin das Kapillar- Aktivatorsystem KA ein oder mehrere Mittel, ausgewählt aus Gingko - Extrakt, Mäusedorn Extrakt, L- Arginin-Hydrochlorid ( z. B. in Mengen wie beschrieben); das Kapillar- Protektorsystem KP ein oder mehrere Mittel, ausgewählt aus Folsäure, Pyridoxinhydrochlorid, Cyanocobalamin, Tocopherylacetat, omega-3- und 6-Fettsäurehaltigen Ölen, und das Kapillar- Energieversorgungssystem KE ein oder mehrere Mittel, ausgewählt aus Nicotinsäure und dessen Derivaten, insbesondere Nicotinsäureamid, Magnesiumoxid, Coffein, Glukose, Q10 aufweist.
   Insbesondere bevorzugt sind Zusammensetzungen, worin das Kapillar Wirksystem K Gingko - Extrakt, Folsäure, Magnesiumoxid aufweist.

Nachfolgend sind einige Beispiele für Mittel der Wirksysteme KA, KP und KE in den dafür jeweils spezifischen Mengen angegeben wie z. B.:
- KA:: Gingko-Extrakt, z. B. entsprechend 5 g Droge Weinblätter-Extrakt, z. B. entsprechend 2 g Droge Rutin: 1000 mg Aescin: 100 mg Roßkatanien-Extrakt: entsprechend 100 mg Aescin Mäusedorn- Extrakt: entsprechend 7 - 11 mg Ruscogenine L-Arginin (z. B. xHcl) entsprechend 250 mg L-Arginin Eisen (z. B. xCarbonat) entsprechend 6 mg Eisen
- KP:: Tocopherylacetat: entsprechend 42 mg Vitamin E

| | |
|---|---|
| Omega-3-Fettsäurehaltiges Öl: | entsprechend mind. 90 mg Omega-3-Fettsäuren; |
| Omega-6-Fettsäurehaltiges Öl: | entsprechend mind. 360 mg Omega-6-Fettsäuren; |
| Folsäure: | 0,2 bis 5 mg, bevorzugt 0,2 bis 0,8 mg, vor allem 0,4 mg; |
| Vitamin B6: | 2,0 bis 25 mg; bevorzugt: 2,0 bis 10 mg, insbesondere bis 6 mg, und ganz besonders 2 mg |
| Vitamin B12: | 0,003 bis 0,6mg, insbesondere bis 0,1 mg, vor allem 0,006 mg; |

Bevorzugte Mengenbereiche sind hier:

| | |
|---|---|
| Folsäure 0,4 - 1,2 mg; | Folsäure : Vitamin B₆ = 1 : 1 - 12 |
| Vitamin B₆ 1,2 - 4,5 mg; | Vitamin B₁₂ = 1 : 0,0025 - 0,0225 |
| Vitamin B₁₂ 0,003 - 0,009 mg; | Vitamin B₁₂ : Vitamin B₆ = 1 : 40 - 500 |

Bei Mischungen dieser Substanzen kann das Verhältnis

| | |
|---|---|
| Folsäure : | Vitamin B₆ = 1 : 5 |
| Folsäure : | Vitamin B₁₂ = 1 : 0,015 |
| Vitamin B₁₂ | Vitamin B₆ = 1 : 333 |

betragen.
- KE:: Nicotinsäureamid: 30 mg
Magnesiumoxid/Carbonat: entsprechend 150 mg Magnesium
Riboflavin: 4 mg
L-Carnithin (500mg), Coffein, Glukose (500mg);

Ganz besonders bevorzugt wird aus der Gruppe KA L- Arginin- Hydrochlorid, z. B. wie insbesondere beschrieben.

Aus der Gruppe KP ist besonders die Kombination Folsäure, Vitamin B6, Vitamin B12 im Mengenverhältnis 1: 5,0:015 geeignet.

Aus der Gruppe KE werden insbesondere Nicotinsäure / dessen Derivate, insbesondere Nicotinsäureamid und Magnesiumsalze oder Kombinationen hiervon gewählt.

In einer ganz besonders geeigneten Ausführungsform wird ein System KA mit zusammen mit einem System KP mit Folsäure, Vitamin B6 oder Vitamin B12 kombiniert.

Eine weitere geeignete Ausführungsform sieht die oben beschriebene Kombination KA, KP zusammen mit Nicotinsäure, dessen Derivate, insbesondere Nicotinsäureamid, Magnesiumsalz, oder Kombinationen hiervon vor.

Die einzelnen Substanzen aus den genannten Gruppen sind für sich bekannt bzw. können nach dem Fachmann bekannten Verfahren hergestellt werden.

### II) Spezifisches Wirksystem S

Wie erläutert, weist die erfindungsgemäße Zusammensetzung neben dem Kapillar- Wirksystem K weiterhin ein spezifisches Wirksystem S, ausgewählt aus Mitteln der Gruppe, umfassend Haut- und Anhangsgebilde (Haare, Nägel) beeinflussende Mittel S1, cerebral wirksame Mittel S2, immunologisch wirksame Mittel S3 , Knochen beeinflussende Mittel S4, Muskelwirksame Mittel S5 , den Gastrointestinaltrakt beeinflussende Mittel S6 oder Augen wirksame Mittel S7 auf. Insbesondere bevorzugt sind hierzu Zusammensetzungen, welche ein Kapillar-Aktivatorsystem KA und ein Kapillar-Protektorsystem KP und ein spezifisches Wirksystem S1 oder spezifisches Wirksystem S2 oder S3; oder solche, welche zusätzlich dazu noch ein Kapillar Energieträgersystem KE aufweisen.

Dem auf dem Gebiet der Herstellung derartiger Zusammensetzungen zur systemischen bzw. enteralen, insbesondere Lebensmittelprodukten tätigen Fachmann sind die für die oben genannten einzelnen Gruppen jeweils geeigneten Wirkstoffe und die für ihren Einsatz geeignete Mengen bekannt und können demnach je nach Wirkziel ausgesucht und in die efindungsgemäße Zusammensetzung inkorporiert werden.

Als bevorzugte Mittel des Wirksystems S können die folgenden Substanzen beispielhaft genannt werden:
Haut- und Anhangsgebilde System S1:
   Retinol, Retinolacetat / -palmitat (Vitamin A), Thiaminnitrat (B₁), Biotin, Ca-pantothenat (B₅) (B- Vitamine), Kieselerde, Kieselsäure, Zinkoxid / -sulfat /-gluconat, pflanzliche Extrakte wie Algen, Papaya, Schachtelhalm, Kamille, Aloe vera, Aminosäuren wie Cystein, Methionin, Gammalinolensäure (GLA), Linolsäure; Vitamin C (Ascorbinsäure, Calcium-ascorbat / Natrium-ascorbat, Kaliumascorbat).
Cerebral - System S2:
   pflanzliche Extrakte wie Ginseng, Paranuß, Wassernabel, Frauenwurz , Aminosäuren wie Glutaminsäure, Tyrosin, Tryptophan, Uridin ; Docosahexaensäure (DHA), Phosphatidylserin , Phosphatidylinositol, Cholin, Lecithin.
Immunologisches System S3:
   Vitamine wie Carotinoide wie Lycopin, Natriumselenat, pflanzliche Extrakte wie Grüntee, Echinacea, Schisandra, Astragalus, Reishi, Shiitake, Maistake, Wiesenklee, Soja, Katzenkralle, Traubenkern, Brokkoli, Tomate, Zwiebel, Blumenkohl, Citrus-Bioflavonoide, Buchweizen, Grapefruit, Aminosäuren wie Threonin, Histidin, Glutathion, Superoxiddismutase.
Skelett- System S4:
   Substanzen aus der Vitamin D Gruppe wie Cholecalciferol, Ergocalciferol, Vitamin K1 (Phyllochinon), Calciumsalze wie Calcium-carbonat / -citrat / -chlorid / - gluconat / -lactat / -oxid, Di-Calciumphosphat / -hydrogenphosphat, Fluoride wie Kaliumfluorid, Natriumfluorid, Spurenelemente wie Kupfercarbonat / -citrat / -lysinkomplex / -sulfat l-gluconat, Mangancarbonat, -chlorid, - citrat, -gluconat,-sulfat, pflanzliche Extrakte wie Sojaisoflavone, Alfalfa, Gerstengras, Weißer Gänsefuß, Aminosäuren wie Lysin.
Muskel - System S5:
   Inositol (B₈), Aminosäuren wie Leucin, Isoleucin, Valin.
Gastrointestinal - System S6:
   α-Linolensäure,
   Kaliumsalze wie Kalium-carbonat / hydrogencarbonat / -citrat / -chlorid / -gluconat / -lactat / -phosphat,
   Chromsalze wie Chromchlorid / -picolinat / -sulfat.
Augen - System S7:
   Carotinoide wie Beta / Alpha-Carotin, Beta-Cryptoxanthin, Lutein, Zeaxanthin, pflanzliche Extrakte wie Rotwein-, Holunder-Bioflavonoide, Quercetin; Aminosäuren wie Taurin.

Es ist auch von besonderem Vorteil, wenn die erfindungsgemäße Zusammensetzung 1 bis 5, bevorzugt 1 bis 3 Verbindungen des Systems KA; 1 bis 5, bevorzugt 1 bis 3, Verbindungen des Systems KP; 1 bis 5, bevorzugt 1 bis 3, Verbindungen des Systems KE; und / oder 1 bis 5, bevorzugt 1 bis 3 Verbindungen des Systems S aufweist.

Besondere bevorzugte Ausgestaltungen erfindungsgemäßer derartiger Systeme sind Zusammensetzungen, worin als Mittel des Systems S1 eine oder mehrere Substanzen, ausgewählt aus Cystein, Methionin, Biotin, Zinkoxid, Thiaminnitrat (B1), Calciumpantothenat (B5), Kieselerde, als Mittel des Systems S2 Ginseng, Paranuß, Frauenwurz, Aminosäuren wie erläutert oder Mischungen hiervon bzw. als Mittel des Systems S3 pflanzliche Extrakte wie Grüntee, Echinacea, Schisandra, Astragalus, Reishi, Schiitake, Maistake, Wiesenklee, Soja, Katzenkralle, Traubenkern, Brokkoli, Tomate, Zwiebel, Blumenkohl, Citrus - Bioflavonoide, Buchweizen, Grapefruit oder als Mittel des Augen - Systems S7 ein oder mehrere aus Carotinoide wie Beta / Alpha-Carotin, Beta-Cryptoxanthin, Lutein, Zeaxanthin, pflanzliche Extrakte wie Rotwein-, Holunder-Bioflavonoide Quercetin; Aminosäuren wie Taurin enthalten sind.

bzw. auch derartige Zusammensetzungen, worin das Aktivatorsystem KA L-Arginin-Hydrochlorid, das Protektorsystem KP Mittel aus der Gruppe KP: Folsäure; Pyridoxin-HCL (B6); Cyanocobalamin (B12); Tocopherol (E); Traubenkernöl, und oder Johannisbeersamenöl und das oder die Mittel der Gruppe KE ausgewählt sind aus Q10, Nicotinsäureamid (Niacin, B3) oder Mischungen hiervon. Weiterhin bevorzugt sind Zusammensetzungen mit einem System KA+ KP oder KA+KE+ KP + S5 oder S6.

Insbesondere bevorzugt sind Zusammensetzungen mit Mitteln der Gruppe S1 oder S2 oder S3, welche 0,1 bis 10% Zusatzstoffe aufweisen.

Die einzelnen Wirkstoffe der Gruppe S, deren Herstellung und deren geeignete Mengen sind an sich bekannt.

Es sind auch pharmakologisch wirksame Wirkstoffe möglich, die den einzelnen Gruppen S1 bis S7 zugeordnet werden können. Beispielhaft können hierzu genannt werden (wobei auch andere bekannte möglich sind):
Haare: Finasterid, Minoxidil
Haut: Isotretinoin, Prednisolon
Cerebrum: Amantadin, Dopamin
Skelett: Calcitonin, Risedronsäure
Muskeln: Dantrolen-Natrium, Tetrazepam
Augen: Acetazolamid, Pilocarpin
Gastro-Intestinaltrakt: Famotidin, Metoclopramid

Die genannten Mittel sind nur beispielhaft und nicht abschließend erwähnt. Der Fachmann kann ggf. die gewünschten Stoffe inkorporieren.

### III. Zusatzstoffe

Die erfindungsgemäße Zusammensetzung kann Zusatzstoffe in einer Menge von 0,1 bis 90 Gew.%, oder bevorzugt 1 bis 90 Gew.%, insbesondere 0,5 bis Gew. 80%, vor allem 2 bis 80 Gew.%, oder 1 bis 50 Gew.%, insbesondere 2 bis 50 Gew.%, bezogen auf das Gesamtgewicht der Zusammensetzung aufweisen, welche aus der Herstellung der genannten Darreichungsformen resultieren. Dazu gehören insbesondere für die enterale, vor allem orale Anwendung: Träger in flüssiger oder fester Form sowie pharmazeutisch zulässige Zusätze oder auch Zusatzstoffe, die für die Herstellung von Supplementen wie Nahrungsergänzungsmittel, ergänzende bilanzierte Diät, Diätikum bekannt sind.

Üblicherweise besteht die Trägergrundlage bei festen oralen Verabreichungsformen (Tablette, Kapsel, Dragee) aus üblichen, auch pharmazeutisch zulässigen, Tablettierhilfsstoffen wie Sprengmittel, Füllstoffe, Fließmittel, ggf. auch Lösehilfen wie Zitronensäure, Hydrogencarbonat (z. B. für Brausetabletten), Flüssigkeiten wie Wasser, (Frucht)Säfte, Kohlenhydrat-Protein- Mischungen (für Riegel) oder z. B. aus Gelatineweichkapseln, in die die Zusammensetzung inkorporiert wird. Besonders konzipierte Darreichungsformen sind hier nicht erforderlich, es werden die jeweils nach dem Stand der Technik bekannten Methoden und Produkte angewendet.

### Herstellung

Die erfindungsgemäße Zusammensetzung zur beschriebenen wie vor allem enteralen Anwendung wird in an sich bekannter Weise hergestellt, indem man das System K, enthaltend KA, KP, KE oder Kombinationen hiervon herstellt durch Mischen der einzelnen gewünschten Substanzen, und dieses sodann mit dem System S, ausgewählt aus S1, S2, S3, S4, S5, S6 oder S7 in geeigneter Weise gegebenenfalls unter Verwendung jeweils geeigneter Zusatzstoffe für die gewünschte orale Verabreichungsform wie Träger, Füllstoffe, Tablettierhilfsmittel wie genannt, auf an sich bekannte Weise vermischt und homogenisiert. Methoden zur Herstellung derartiger Lebensmittel oder auch pharmazeutischer Mittel sind z. B. in Lehrbüchern beschrieben und allgemein bekannt.

Je nach Wirkstoff der Gruppe S können so pharmazeutische Zubereitungen oder auch Supplemente wie beschrieben erhalten werden.

Die Zusammensetzung kann insofern auch Teil eines Mittels in Form einer Zubereitung wie essbare Riegel etc. sein. Hierzu können Nahrungskomponenten, ausgewählt aus Aminosäuren, Kohlenhydraten, Fetten, die für die menschliche oder tierische Nahrung geeignet sind, z. B. Kohlenhydrat - Protein- Mischungen, gewählt werden. Bekannte Einzelkomponenten sind z. B. Fruchtsaft, Nektar, Fruchtmus wie Apfelsaft, Orangensaft, Apfelmus. Weiterhin bekannte Einzelkomponenten sind Getreideprodukte wie Weizen- Roggen-, Hafermehl, Maissirup, Milcheiweiß, Molke, Lecithin, Milchzucker. Je nach Wahl derartiger Vertreter der genannten Einzelkomponenten, können wie erwähnt Riegel, Flüssigkeiten, Drinks, z. B. Kraftdrinks (vorzugsweise nicht sirupös/ wässrig sirupös), Brausetablette oder auch bilanzierte diätetische Ergänzungsmittel dieser Art hergestellt werden.

Dazu wird die wie beschrieben bereitete Zusammensetzung mit einer oder mehrere Nahrungskomponenten auf an sich bekannte Weise gemischt, und die gewünschte Form erhalten durch übliche Herstellungsverfahren.

### Anwendung

Die erfindungsgemäßen Zusammensetzungen werden insbesondere für die systemische bzw. enterale Anwendung konzipiert. Dabei sind orale Verabreichungsformen von Zusammensetzungen aus dem Aktiv- Wirksystem K + System S besonders geeignet.

Besonders geeignete Verabreichungsformen sind Gelatinekapseln, z. B. Weichgelatinekapsel, sowie Tabletten.

Aufgrund der besonderen Kombination der erfindungsgemäßen Zusammensetzung werden insgesamt stabile Produkte erhalten, deren Wirkungsweise unabhängig von der Darreichungsform erzielt werden kann.

Insbesondere kann diese Zusammensetzung bei strukturellen Veränderungen und funktionellen Störungen der betreffenden Organe, insbesondere des Haares, der Nägel, der Haut, der Augen, Muskeln, des Gastrointestinaltrakts, der cerebralen Fähigkeiten, des Skelettes, des immunologischen Systems als Supplement (nicht therapeutisch) wie Nahrungsergänzungsmittel, Diätikum, ergänzende bilanzierte Diät oder als pharmazeutisches Mittel bzw. zur Herstellung eines pharmazeutischen Mittels eingesetzt werden.

Diese Zusammensetzung kann wie erwähnt eingesetzt werden zur systemischen, insbesondere oralen Behandlung oder Prävention von strukturellen Veränderungen und funktionellen Störungen der betreffenden Zielorgane wie Haut, Nägel, Haare, Cerebrum, Skelett, Muskeln, Augen und des immunologischen Systems, Gastrointestinaltrakt von Säugern, insbesondere Menschen.

Zur Herstellung von Kapseln oder Tabletten werden hierfür gebräuchliche Hilfsstoffe wie Lactose, Saccharose, Sorbit, Talkum, Stearinsäure, Magnesiumstearat, Gummi arabicum, Kartoffelstärke, Gelatine, PVP, Glycerin, Hydroxypropylmethylcellulose, Maltodextrin, Konservierungsmittel sowie übliche Materialien für Überzüge wie PEG 6000, Maisstärke, Zucker, Talkum, Farbstoffe in an sich bekannter Weise eingesetzt. Wenn per os verabreicht wird, kann die beschriebene Zusammensetzung z. B. vermischt werden mit Lactose, Saccharose, Stärkepulver, Celluloseester oder Alkansäuren, Cellulosealkylester, Talk, Stearinsäure, Magnesiumstearat, Magnesiumoxid, Natrium- und Calciumsalzen von Phosphor- und Schwefelsäuren, Gelatine, Akaziengummi, Natriumalginat, Glykole, Polyvinylpyrrolidon und/oder Polyvinylalkohol und dann tablettiert oder verkapselt werden. Die Zusatzstoffe und Tablettierungsverfahren sind allgemein bekannt und z.B. im aktuellen Europäischen Arzneibuch beschrieben. Insbesondere können sich die strukturellen Veränderungen und funktionelle Störungen auf Schwächezustände z. B. nach Krankheiten oder besonderen Belastungen (Stress), Alterserscheinungen oder auch Mangelerscheinungen beziehen.

Die Menge des verabreichten Mittels und der Dosierungsplan zur Prävention oder Behandlung eines wie oben beschriebenen Zustandes mit der erfindungsgemäßen Zusammensetzung hängen bei einer medizinischen Indikation von einer Vielzahl von Faktoren ab, einschließlich dem Alter, Gewicht, Geschlecht und Zustand des Patienten, der Schwere der Störung, der Verabreichungsroute und der jeweiligen verwendeten Verbindung, und kann deshalb weitestgehend schwanken. Die erforderliche oder gewünschte Dosis kann auf ein- oder mehrere Male täglich, insbesondere 1 bis 3 mal verteilt werden.

Bei der Verabreichung als Supplement werden hierfür allgemein übliche Mengen vorgeschlagen, welche auf ein- bis mehrmals, insbesondere 1 bis 3 mal oder 1 bis 2 mal täglich verteilt werden können. Diese können in fester Form, z. B. als Tablette, Kapsel, Dragee, oder als Riegel bzw. eine entsprechende Menge gelöst in Säften vorliegen.

### Beispiele

Die Erfindung wird anhand der nachfolgenden Beispiele 1 bis 4 näher erläutert. Die Wirksamkeit der erfindungsgemäßen Zusammensetzung wird anhand des nachfolgenden Beispiels 5 gezeigt.

### Beispiel 1

### Dragee mit Wirksystem S1 (Haut)

Durch Vermischen der nachfolgenden Substanzen und Verpressen unter üblichen Bedingungen wurde ein erfindungsgemäßes Dragee erhalten aus:
I. Kapillar- Wirksystem K:
   1) Mittel aus der Gruppe KA: L-Arginin-Hydrochlorid:
   2) Mittel aus der Gruppe KP: Folsäure; Pyridoxin-HCL (B6); Cyanocobalamin (B12); Tocopherol (E); Traubenkernöl:
   3) Mittel aus der Gruppe KE: Q10:
II: Spezifisches Wirksystem S
   S1:Thiaminnitrat (B1), Calciumpantothenat (B5), Kieselerde,
III: Zusatzstoffe: Sojalecithin, Sojaöl, Gelatine, Glycerin

### Beispiel 2

### Tablette, mit Wirksystem S1 (Haare)

Durch Vermischen der einzelnen Inhaltsstoffe und Zusatzstoffe auf bekannte Weise wurde eine Tablette wie folgt erhalten:
I. Kapillar- Wirksystem K
   1) KA: L-Arginin-Hydrochlorid
   2) KP: Johannisbeersamenöl
   3) KE: Nicotinsäureamid (Niacin, B3)
II: Spezifisches Wirksystem S
   S1: Cystein, Methionin, Biotin, Zinkoxid
III. Zusatzstoffe: Lactose, Magnesiumstearat, Aerosil

### Beispiel 3

### Hartgelatinekapsel mit Wirksystem S3 (Skelett)

Auf bekannte Weise wurde eine Hartgelatinekapsel mit folgenden Substanzen erhalten:
I. Kapillar- Wirksystem K
   KA: L-Arginin-Hydrochlorid
II: Spezifisches Wirksystem S
   S3: Cholecalciferol (D3), Phyllochinon (K1), Calciumcarbonat, Kupfercarbonat III. Zusatzstoffe: Gelatine, Magnesiumstearat, Talkum, Aerosil.

### Beispiel 4

### Weichgelatinekapsel, Wirksystem S1

Durch Vermischen der einzelnen Inhaltsstoffe auf bekannte Weise wurde eine Weichgelatinekapsel wie folgt erhalten:
I. Kapillar- Wirksystem K
   KA: L-Arginin-Hydrochlorid;
   KP: Vitamin E, Traubenkernöl, Leinöl;
   KE: Coenzym Q10; Magnesiumsalz; Nicotinsäureamid;
II. Vitamin A Palmitat; Biotin; Zinksalz; Vitamin C Ascorbat.
III. Zusatzstoffe: Glycerin, Gelatine, Sorbit.

### Beispiel 5

### Anwendungsbeispiel

60 freiwillige ausgesuchte hautgesunde Probandinnen, je 15 pro Gruppe, erhielten über einen Zeitraum von 6 Monaten eine Vitalstoffkombination wie in der nachfolgenden Tabelle beschrieben, bzw. eine Kombination, enthalten nur die Kombination K bzw. S gemäß Tabelle bzw. Placebo (Gruppe K+S, K, S, Kontrolle) täglich morgens und abends eine Weichgelatinekapsel, mit den unten angegebenen Substanzen.

Die Überlegenheit der Kombination eines spezifischen Wirksystems "S" mit einem Kapillar-Wirksystem "K" läßt sich anhand dieses Anwendungsbeispiels eindrucksvoll zeigen. Bei diesem Verfahren wird bei freiwilligen Probanden ein Hautareal mit ultraviolettem Licht bestrahlt und anschließend die Photonenemission aus der bestrahlten Haut mittels "Single Photon Counting" gemessen. Je geringer die Photonenemission um so besser der Schutz der Haut vor oxidativen Schäden. Nach Einnahme der jeweiligen untersuchten Zusammensetzung K, bzw. S bzw. K+S wird ein Effekt erreicht, der sich UV-Licht- protektiv messbar nachweisen ließ. Die schützenden Eigenschaften sind bei der Kombination K+S überraschender Weise ausgeprägter als bei den Einzelkomponenten K, S und der Kontrollgruppe. Diese Tatsache dient als Wirksamkeitsnachweis für die überlegene Formulierung gemäß der erfindungsgemäßen, auch als Micro Nutri-Targeting- System bezeichenbaren Kombination.

### Methodenbeschreibung:

Zu Beginn und am Ende der Einnahme der jeweiligen Zusammensetzung wurde auf dem Rücken vor Studienbeginn ausgesuchter hautgesunder Probandinnen die sogenannte ICL-S (induced chemiluminescence of human skin) gemessen. Hierbei wird eine Kuppel aus Flüssigkeits-Lichtwellenleitern mit einem zentral angeordneten Lichtwellenleiter zur Bestrahlung in einem optimierten Abstand und Winkel zur Haut lokalisiert, ohne diese zu berühren. Die Lichtwellenleiter befinden sich in einem Messkopf, der fest auf die Haut gedrückt wird. Diese Anordnung erlaubt einen definierten Photonentransfer von der Haut zum Detektor und kompensiert Einflüsse wie Schwitzen oder Bewegungen der Testperson.

Je Person wurden vier Untersuchungsareale zwei Minuten mit UVA (320 ≤ λ ≤ 400 nm) bestrahlt (Bestrahlungsenergie 10mW/cm², λmax = 350 nm). Unmittelbar nach diesem UV-Stress wurde die Photonenemission der Haut mittels Single-Photon-Counting über einen Zeitraum von 200 s gemessen. Die Menge an freigesetzten Photonen korreliert dabei mit dem durch UVA-Strahlung ausgelösten "oxidativen Stress" [Sauermann G, Mei WP, Hoppe U, Stäb F: Ultraweak photon emission in vivo: Influence of topically applied antioxidants on human skin. Mehtods Enzymol 199; 300:419-428].

Der Mittelwert der integralen Photonenemission sowie die mittleren Abklingkinetiken der von der Haut emittierten Photonen wurden vor und nach Einnahme der Kombination berechnet und auf signifikante Unterschiede getestet.

Unterschiede im Hautkolorit wurden rechnerisch berücksichtigt.

Im Rahmen der vorliegenden Untersuchung konnte belegt werden, dass die Intervention mit der erfindungsgmäßen Kombinationen K +S zu einer Stärkung der antioxidativen Schutzfunktionen der Haut führen. Hierbei wurden nicht, wie sonst üblich, oxidierte Metabolite indirekt nachgewiesen, es erfolgte vielmehr eine direkte Messung der UV-induzierten Chemilumineszenz der Haut bei den einzelnen Probandinnen. Auf diese Weise wurden mittels einer physikalischen Messmethodik funktionell relevante Ergebnisse erhalten, die einen unmittelbaren Rückschluss auf den Schutz der Haut gegen die von exogenen Noxen ausgehenden Schäden erlauben. Diese Verbesserung der Schutzfunktion der Haut ist nicht nur wesentlich im Sinne eines dermalen Anti-Aging-Effektes, sondern auch im Sinne der Prävention vieler altersabhängiger Erkrankungen der Haut, die mit oxidativen Belastungen zusammenhängen.

Da infolge der UVA-Belastung durch Sonnenstrahlen Schäden der mitochondrialen DNA auftreten, die nicht nur über Jahre bestehen bleiben können, sondern deren Gehalt in der Haut durch einen einmal induzierten Mechanismus auch ohne weitere Bestrahlung weiter ansteigen kann, werden Antioxidantien zur Vermeidung von Photo-Aging im Sinne einer Dauerbehandlung und nicht nur für die Zeit der Sonnenexposition empfohlen [GD-Symposium: Wirkungen von Dermakosmetika. Dermotopics 1, 2002; 93:9-12].

In keinem Fall sollte allerdings die Verwendung von Antioxidantien zu einem übermäßigen Sonnengenuß verleiten.

Die jeweils eingesetzte Kombination K, bzw. S bzw. K+S enthielt folgende Substanzen (Mikronährstoffe) nach Art und Menge wie aufgeführt:

| Modul | Substanz (Mikronährstoff) | Tagesdosis |
|---|---|---|
| K | Kapillar-Aktivatorsystem KA Gefäß-Erweiterung, Blutfluss-Steigerung | |
| | L - Arginin | 250 mg |
| Kapillar-Wirk-System | Kapillar-Protektorsystem KP Endothelstablisierung, Gefäßschutz, Lipoproteinschutz | |
| | Vitamin E | 42 mg |
| | Traubenkernöl | 514 mg |
| | enthält mind. Ω-6-Fettsäuren | 360 mg |
| | Leinöl | 178,8 mg |
| | enthält mind. Ω-3-Fettsäuren | 90,0 mg |
| | Kapillar-Energieversorgungssystem KE Redoxsysteme und Cofaktoren bei der Energiebereitstellung, Energieträger | |
| | Coenzym Q₁₀ | 5 mg |
| | Magnesium | 300 mg |
| | Niacin | 39 mg |

| S Spezifisches Wirk-System | Spezifisches Wirksystem S Spezifisches Wirksystem Haut S | |
|---|---|---|
| | Vitamin A | 0,8 mg |
| | Biotin | 0,18 mg |
| | Zink | 9,0 mg |
| | Vitamin C | 100 mg |

Die erhaltenen ICL- Werte sind in Figur 1 in Abhängigkeit von der Zeit dargestellt.

Wie hieraus ersichtlich ist, führt die erfindungsgemäße Vitalstoff-Kombination aus K +S zu einer überraschenden Stärkung der antioxidativen Schutzfunktionen der Haut. Dabei zeigte sich unerwarteter Weise ein überadditiver Effekt. Dies ist umso vorteilhafter, da die infolge der UVA-Belastung durch Sonnenstrahlen aufgetretenen Schäden der mitochondrialen DNA über Jahre bestehen bleiben können und darüber hinaus auch deren Gehalt in der Haut durch einen einmal induzierten Mechanismus auch ohne weitere Bestrahlung weiter ansteigen kann. Antioxidative Mittel sind daher besonders wünschenswert zur Vermeidung von Photo-Aging, insbesondere auch im Sinne einer Dauerbehandlung und nicht nur für die Zeit der Sonnenexposition.

Die Erfindung umfasst daher Zusammensetzungen mit einer anwendungsbezogenen Wirkstoffdifferenzierbarkeit, welche gekennzeichnet sind dadurch, dass sie I.) ein Kapillar- Wirksystem K aufweisen, das ein oder mehrere Systeme umfasst, die ausgewählt sind aus einem a) Kapillar - Aktivatorsystem KA, umfassend ein oder mehrere Mittel, die ausgewählt sind aus I) KA 1) Blutfluss steigernden Mitteln; KA 2), Gefäßwand stabilisierenden Mitteln; KA 3) Gefäß erweiternden Mitteln; und / oder einem b) einem Kapillar-Protektor-System KP, umfassend ein oder mehrere Mittel, die ausgewählt sind aus einer oder mehreren Gruppen aus KP 1) Mitteln zur Endothelstabilisierung; KP 2) Mitteln zum Lipoproteinschutz; KP 3) Mitteln zur Leukozyten- / Thrombozytenstabilisierung; und / oder einem c) Kapillar - Energieversorgungssystem KE, umfassend ein oder mehrere Mittel, die ausgewählt sind aus einer oder mehreren Gruppen aus KE 1) Redoxsystemen bei der Energiebereitstellung;KE 2)Cofaktoren bei der Energiebereitstellung; KE 3) Energieträgern; und II.) ein spezifisches Wirksystem S, ausgewählt aus Mitteln der Gruppe, umfas-send Haut- und -Anhangsgebilde beeinflussende Mittel S1, cerebral wirksame Mittel S2, immunologisch wirksame Mittel S3, Knochen beeinflussende Mittel S4, Muskelwirksame Substanzen S5, den Gastrointestinaltrakt beeinflussende Substanzen S6 oder das Auge beeinflussende Mittel S7, sowie III.) 0,1 bis 90% Zusatzstoffe enthalten.

Ferner umfasst die Erfindung derartige Zusammensetzungen, worin das Kapillar-Wirksystem K ein Kapillar- Aktivatorsystem KA, oder ein Kapillar- Protektorsystem KP, oder ein Kapillar- Energieversorgungssystem KE aufweist; oder solche Zusammensetzungen, die ein Kapillar- Aktivatorsystem KA und ein Kapillar-Protektorsystem KP; oder die weiterhin ein Kapillar - Energieversorgungssystem KE aufweisen.

Ferner umfasst sind derartige Zusammensetzungen, die ein Kapillar- Aktivatorsystem KA und ein Kapillar-Protektorsystem KP und ein spezifisches Wirksystem S1 oder spezifisches Wirksystem S2 oder S3; oder zusätzlich dazu noch ein Kapillar Energieträgersystem KE enthalten.

Ferner umfasst sind derartige Zusammensetzungen, worin das Kapillar- Aktivatorsystem KA ein oder mehrere Mittel, ausgewählt aus Gingko - Extrakt, Mäusedorn Extrakt, L- Arginin-Hydrochlorid; das Kapillar- Protektorsystem KP ein oder mehrere Mittel, ausgewählt aus Folsäure, Pyridoxinhydrochlorid, Cyanocobalamin, Tocopherylacetat, omega-3- und -6-Fettsäurehaltigen Ölen, und das Kapillar-Energieversorgungssystem KE ein oder mehrere Mittel, ausgewählt aus Nicotinsäure und dessen Derivaten wie insbesondere Nicotinsäureamid, Magnesiumoxid, Co Enzym Q10, Coffein, Glukose aufweisen.

Ferner umfasst sind derartige Zusammensetzungen, worin das Kapillar Wirksytem K Gingko - Extrakt, Folsäure, Magnesiumoxid aufweist.

Ferner umfasst sind derartige Zusammensetzungen, worin als Mittel des Systems S1 eine oder mehrere Substanzen, ausgewählt aus Cystein, Methionin, Biotin, Zinkoxid, Thiaminnitrat (B1), Calciumpantothenat (B5), Kieselerde, als Mittel des Systems S2 Ginseng, Paranuß, Frauenwurz, Aminosäuren wie Glutaminsäure, Tyrosin, Tryptophan, Uridin oder Mischungen hiervon bzw. als Mittel des Systems S3 pflanzliche Extrakte wie Grüntee, Echinacea, Schisandra, Astragalus, Reishi, Schiitake, Maistake, Wiesenklee, Soja, Katzenkralle, Traubenkern, Brokkoli, Tomate, Zwiebel, Blumenkohl, Citrus-Bioflavonoide, Buchweizen, Grapefruit oder als Mittel des Augen - Systems S7 ein oder mehrere aus Carotinoide wie Beta / Alpha-Carotin, Beta-Cryptoxanthin, Lutein, Zeaxanthin, pflanzliche Extrakte wie Rotwein-, Holunder-Bioflavonoide Quercetin; Aminosäuren wie Taurin; enthalten sind.

Ferner umfasst sind derartige Zusammensetzungen, die für galenische oder pharmazeutische Formulierungen übliche Zusatzstoffe in einer Menge von 0,1 bis 90 Gew.%, insbesondere 2-80% Gew.% aufweisen.

Ferner umfasst sind derartige Zusammensetzungen, die systemisch, insbesondere enteral applizierbar ist.

Ferner umfasst sind derartige Zusammensetzungen, die in einer für die systemisch bzw. orale Anwendung geeigneten Verabreichungsform vorliegen.

Ferner umfasst sind derartige Zusammensetzungen, die in Form einer Tablette, Kapsel, eines Dragees, einer Flüssigkeit oder eines Riegels, Brausetablette, Gelatinekapseln, in einer geeigneten Trägergrundlage, ausgewählt aus Tablettierhilfsstoffen, Wasser, Fruchtsäften, Kohlenhydrat-Protein-Mischungen vorliegt. Ferner umfasst ist die Verwendung derartiger Zusammensetzungen als Supplement bei strukturellen Veränderungen und funktionellen Störungen der betreffenden Zielorgane, insbesondere Haare, Nägel, Haut, Cerebrum, Skelett, Muskeln, Augen, des immunologischen Systems und des Gastrointestinaltrakts.

Ferner umfasst ist die Verwendung derartiger Zusammensetzungen, wobei das Supplement ein Nahrungsergänzungsmittel, ein Diätikum, ergänzende bilanzierte Diät ist.

Ferner umfasst ist die Verwendung derartiger Zusammensetzungen, wobei sich die strukturellen Veränderungen und funktionellen Störungen auf Schwächezustände, Alterserscheinungen oder Mangelerscheinungen beziehen.

Ferner umfasst ist die Verwendung derartiger Zusammensetzungen, wobei das Supplement in Form eines Dragees, einer Kapsel, einer Tablette, eines Getränks, eines Riegels vorliegt.

Ferner umfasst ist die Verwendung derartiger Zusammensetzungen zur Herstellung eines Mittels in Form eines Dragees, Kapsel, einer Tablette, eines Getränks, eines Riegels zur Beeinflussung von strukturellen Veränderungen und funktionellen Störungen, insbesondere Schwächezuständen und Alterserscheinungen, des Haares, Nägel, der Haut, der cerebralen Fähigkeiten, des Skelettes, des immunologischen Systems, der Muskeln, des Gastrointestinaltrakts oder auch der Augen; sowie eine derartige Verwendung als pharmazeutisches Mittel.

## Patentansprüche

1. Systemisch applizierbare Zusammensetzung mit einer anwendungsbezogenen Wirkstoffdifferenzierbarkeit, **dadurch gekennzeichnet, dass** sie
I.) ein multidifferenzierbares Modul (Kapillar-Wirksystem K), umfassend ein
a) agonistisches Kapillar - Aktivatorsystem KA, umfassend ein oder mehrere Mittel, ausgewählt aus Weinblätter-Extrakt, Rutin, Aescin, Roßkastanien-Extrakt, Mäusedorn-Extrakt, Eisen in einer Menge entsprechend 6 mg Eisen, L-Arginin, Ginkgo-Extrakt; und /oder
b) ein antagonistisches Kapillar-Protektorsystem KP, umfassend ein oder mehrere Mittel, ausgewählt aus Tocopherol / -acetat (Vitamin E), Anthocyane, α-Liponsäure, Folsäure (B₉), Pyridoxin HCL (B₆), Cyanocobalamin (B₁₂), Lieferanten für Ω-3- / Ω-6-Fettsäuren (Borretschöl, Leinöl, Traubenkernöl, schwarzes Johannisbeersamenöl, Nachtkerzenöl, Lachsöl, Schwarzkümmelöl); und / oder
c) ein agonistisches Kapillar - Energieversorgungssystem KE, umfassend ein oder mehrere Mittel, ausgewählt aus Nicotinsäure (Niacin), Nicotin säureamid (B₃), Magnesiumoxid / -citrat / -carbonat / -chlorid / gluconat / -phosphat / -lactat/-sulfat, Riboflavin (B₂), L- Carnitin, Q₁₀, Coffein, Glucose;
und
II.) ein spezifisches Wirksystem S, ausgewählt aus Mitteln der Gruppe, umfassend Haut- und -Anhangsgebilde beeinflussende Mittel S1, cerebral wirksame Mittel S2, immunologisch wirksame Mittel S3, Knochen beeinflussende Mittel S4, Muskelwirksame Substanzen S5, den Gastrointestinaltrakt beeinflussende Substanzen S6 oder das Auge beeinflussende Mittel S7, aufweist sowie
III.) 2 bis 80 Gew. % Zusatzstoffe enthält.

2. Zusammensetzung gemäß Anspruch 1, **dadurch gekennzeichnet, dass** sie ein System KP und ein System KE aufweist.

3. Zusammensetzung gemäß einem der Ansprüche 1 oder 2, **dadurch gekennzeichnet, dass** das System KP Folsäure, Cyanocobalamin, Pyridoxin HCL (B₆), Tocopherylacetat oder Kombinationen hiervon; und das System KE Nicotinsäure, Nicotinsäureamid und Magnesiumsalze oder Kombinationen hiervon aufweist.

4. Zusammensetzung gemäß einem der Ansprüche 1 bis 3, **dadurch gekennzeichnet, dass** sie Eisencarbonat / -fumarat / -diphosphat / -lactat / -saccharat / -gluconat / -sulfat / -citrat, in einer Menge entsprechend 6 mg Eisen (allein oder in Kombination) aufweist.

5. Zusammensetzung gemäß Anspruch 1 bis 4, **dadurch gekennzeichnet, dass** sie als Wirkstoffe des Systems S ein oder mehrere Mittel aus der Gruppe S1, umfassend Retinol, Retinolacetat / palmitat (Vitamin A), Thiaminnitrat (B₁), Biotin, Ca-pantothenat (B₅) (B- Vitamine), Kieselerde, Kieselsäure, Zinkoxid / - sulfat /-gluconat, pflanzliche Extrakte wie Algen, Papaya, Schachtelhalm, Kamille, Aloe vera, Aminosäuren wie Cystein, Methionin, Gammalinolensäure (GLA), Linolsäure; Vitamin C (Ascorbinsäure, Calcium-ascorbat / Natrium-ascorbat, Kaliumascorbat) aufweist.

6. Zusammensetzung gemäß einem der Ansprüche 1 bis 5, **dadurch gekennzeichnet, dass** das Mittel des spezifischen Wirksystems S ausgewählt ist aus einem oder mehreren Substanzen, ausgewählt aus Vitamin A Palmitat; Biotin; Zinksalz; Vitamin C Ascorbat.

7. Zusammensetzung gemäß einem der Ansprüche 1 bis 6, **dadurch gekennzeichnet, dass** sie in einer für die orale Anwendung geeigneten Verabreichungsform vorliegt.

8. Verwendung einer Zusammensetzung gemäß einem der Ansprüche 1 bis 7 zur Herstellung von Mitteln in Form einer Tablette, Kapsel, eines Dragees, einer Flüssigkeit oder eines Riegels, Brausetablette, in einer geeigneten Trägergrundlage, ausgewählt aus Tablettierhilfsstoffen, Wasser, Fruchtsäften, Kohlenhydrat-Protein-Mischungen.

9. Verwendung gemäß Anspruch 8 zur Herstellung von Mitteln in Form von Supplementen (Nahrungsergänzungsmitteln, Ergänzende bilanzierte Diät, Diätetikum), oder pharmazeutischen Mitteln.

10. Verwendung einer Kombination aus einem agonistischen Kapillar - EnergieVersorgungssystem KE und einem antagonistischen Kapillar-Protektorsystem KP als multidifferenzierbares Modul für einen verbesserten und rationaleren Wirkstofftransport zum Zielorgan zur Herstellung von / in Zusammensetzungen mit (speziellen) Wirkstoffen des spezifischen (Zielorgan-) Wirksystems S für Haut/Haare, Nägel, Muskel, Knochen, Verdauung, Auge, Immunsystem oder Gehirn gemäß Anspruch 1, wobei
b) das Kapillar-Protektorsystem KP eine oder mehrere Substanzen, ausgewählt aus Tocopherol / -acetat (Vitamin E), Anthocyane, α-Liponsäure, Folsäure (B₉), Pyridoxin HCL (B₆), Cyanocobalamin (B₁₂), Lieferanten für Ω-3-und Ω-6-Fettsäuren (Traubenkernöl, Johannisbeersamenöl), umfasst;
c) das Kapillar - Energieversorgungssystem KE ein oder mehrere Mittel umfasst, die ausgewählt sind aus Nicotinsäure (Niacin) und dessen geeignete Derivate wie insbesondere Nicotinsäureamid (B₃), Magnesiumoxid / -citrat / -carbonat / -chlorid / gluconat / -phosphat / -lactat / -sulfat, Riboflavin (B₂), L- Carnitin, Q₁₀, Coffein, Glucose.

11. Verwendung gemäß Anspruch 10, **dadurch gekennzeichnet, dass** das System KP Folsäure, Cyanocobalamin, Pyridoxin HCL (B₆), Tocopherylacetat oder Kombinationen hiervon; und das System KE Nicotinsäure, Nicotinsäureamid und Magnesiumsalze oder Kombinationen hiervon aufweist.

12. Verwendung gemäß Anspruch 11, **dadurch gekennzeichnet, dass** das Mittel des spezifischen Wirksystems S ausgewählt ist aus einem oder mehreren Substanzen, ausgewählt aus Vitamin A Palmitat; Biotin; Zinksalz; Vitamin C Ascorbat.

13. Verwendung gemäß einem der Ansprüche 10 bis 12, **dadurch gekennzeichnet, dass** das Modul weiterhin ein agonistisches System KA, ausgewählt aus Ei sen in einer Menge entsprechend 6 mg Eisen aufweist.

14. Verwendung gemäß einem der Ansprüche 10 bis 13, **dadurch gekennzeichnet, dass** die Zusammensetzung je 1 bis 5, vorzugsweise 1 bis 3 Verbindungen des Systems KP, KE, S bzw. KA aufweist.

15. Verwendung gemäß einem der Ansprüche 10 bis 14, **dadurch gekennzeichnet, dass** die Zusammensetzungen in Form einer Tablette, Kapsel, eines Dragees, einer Flüssigkeit oder eines Riegels, Brausetablette, in einer geeigneten Trägergrundlage, ausgewählt aus, Tablettierhilfsstoffen, Wasser, Fruchtsäften, Kohlenhydrat-Protein-Mischungen vorliegt.

16. Verwendung gemäß einem der Ansprüche 10 bis 15 zur Herstellung von /in Zusammensetzungen in Form von Supplementen (Nahrungsergänzungsmitteln, Ergänzende bilanzierte Diät, Diätetikum), oder pharmazeutischen Mitteln.

17. Verwendung gemäß Anspruch 16 zur selektiven Beeinflussung von strukturellen Veränderungen und funktionellen Störungen, insbesondere Schwächezuständen und Alterserscheinungen, des Haares, Nägel, der Haut, der cerebralen Fähigkeiten, des Skelettes, des immunologischen Systems, der Muskeln, des Gastrointestinaltrakts oder auch der Augen; sowie eine derartige Verwendung als pharmazeutisches Mittel.
